(19) Europäisches Patentamt
European Patent Office
Office européen des brevets

(11) EP 0 908 170 B1

(12) # FASCICULE DE BREVET EUROPEEN

(45) Date de publication et mention de la délivrance du brevet:
**31.05.2000 Bulletin 2000/22**

(51) Int Cl.[7]: **A61K 7/00**, A61K 7/48

(21) Numéro de dépôt: **98402250.9**

(22) Date de dépôt: **11.09.1998**

(54) **Emulsion E/H/E stable et son utilisation comme composition cosmétique et/ou dermatologique**

Stabile Wasser-in-Öl-in-Wasser Emulsion und ihre Verwendung als kosmetische und/oder dermatologische Zusammensetzung

Stable water-in-oil-in-water emulsion and its use as cosmetic and/or dermatological composition

(84) Etats contractants désignés:
**AT BE CH DE ES FR GB IT LI NL SE**

(30) Priorité: **03.10.1997 FR 9712364**

(43) Date de publication de la demande:
**14.04.1999 Bulletin 1999/15**

(73) Titulaire: **L'OREAL**
**75008 Paris (FR)**

(72) Inventeurs:
- **Afriat, Isabelle**
  **75014 Paris (FR)**
- **Chanvin, Florence**
  **97450 Soisy/S/Seine (FR)**
- **Guiramand, Carole**
  **91310 Limas (FR)**

(74) Mandataire: **Tezier Herman, Béatrice**
**L' OREAL,**
**D.P.I.,**
**6, rue Bertrand Sincholle**
**92585 Clichy Cédex (FR)**

(56) Documents cités:
**EP-A- 0 750 899**
**EP-A- 0 779 071**
**EP-A- 0 832 645**
**US-A- 5 567 426**

EP 0 908 170 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention se rapporte à une composition se présentant sous forme d'une émulsion triple E/H/E, et à ses applications dans les domaines cosmétique et dermatologique, notamment pour la libération contrôlée d'actifs, plus particulièrement d'actifs sensibles à l'eau et/ou à l'oxygène et d'actifs acides, notamment en vue de nettoyer et/ou traiter et/ou protéger la peau et/ou les muqueuses et/ou les fibres kératiniques.

Il est connu d'introduire dans les compositions cosmétiques et/ou dermatologiques des actifs en vue d'apporter des traitements spécifiques à la peau, par exemple pour lutter contre le dessèchement, le vieillissement ou la pigmentation de la peau, pour traiter l'acné ou certaines maladies de la peau (eczéma, psoriasis), pour combattre les surcharges pondérales, pour favoriser la restructuration de la peau ou son renouvellement cellulaire, ou aussi pour colorer la peau.

Par exemple, l'acide ascorbique (ou vitamine C) est connu pour stimuler la croissance du tissu conjonctif, et notamment celle du collagène. Il permet aussi de renforcer les défenses du tissu cutané contre les agressions extérieures telles que le rayonnement ultraviolet ou la pollution. Il est également utilisé pour enlever les taches et la pigmentation de la peau, et aussi pour favoriser la cicatrisation de la peau.

En outre, il est connu que l'application d'hydroxyacides sur la peau permet de lutter notamment contre le vieillissement cutané et contre certains désordres de la peau tels que l'acné.

Par ailleurs, les émulsions triples sont connues pour présenter un intérêt potentiel pour la libération contrôlée d'actifs. Elles permettent notamment de protéger les actifs sensibles contre les agents extérieurs néfastes pour la stabilité de ces actifs, tels que l'eau ou l'oxygène. Les émulsions eau/huile/eau (E/H/E) sont particulièrement intéressantes du fait que les phases aqueuses interne et externe sont séparées par une couche huileuse. Un actif encapsulé dans la phase interne peut donc être maintenu en dehors de la phase externe (voir article de DAHMER TAGAWA 19th IFSCC Congress Sydney 1996). Ainsi, le document EP-A-779071 décrit une émulsion E/H/E contenant un actif sensible à l'eau.

Malheureusement, certains actifs, et en particulier les actifs acides du fait de leur acidité, déstabilisent les émulsions triples les contenant, en particulier quand la phase aqueuse externe est gélifiée.

Ce manque de stabilité se traduit par une séparation de phases et/ou l'obtention d'une émulsion H/E simple au lieu d'une émulsion triple.

Il subsiste donc le besoin d'une émulsion E/H/E stable, susceptible de contenir des actifs cosmétiques et/ou dermatologiques acides.

La demanderesse a maintenant trouvé de manière inattendue une association de polymères, apte à gélifier la phase aqueuse externe de l'émulsion triple et permettant d'obtenir une émulsion triple eau/huile/eau (E/H/E) stable même en présence d'actifs acides.

Aussi, la présente invention a pour objet une émulsion triple eau/huile/eau, comportant une phase aqueuse externe gélifiée, une phase huileuse constituant avec une phase aqueuse interne une émulsion primaire eau/huile, caractérisée en ce que la phase huileuse comprend un émulsionnant choisi parmi les alkyldiméthicone copolyols et les diméthicone copolyols et en ce que la phase aqueuse externe contient :

1) au moins un copolymère émulsionnant constitué d'une fraction majoritaire d'un monomère acide carboxylique monooléfiniquement insaturé en $C_3$-$C_6$ ou de son anhydride et d'une fraction minoritaire de monomère ester gras d'acide acrylique, et

2) au moins un poly-(acide acrylamidométhyl propane sulfonique) réticulé comprenant, distribués de façon aléatoire :

a) de 90 à 99,9% en poids de motifs de formule générale (III) suivante :

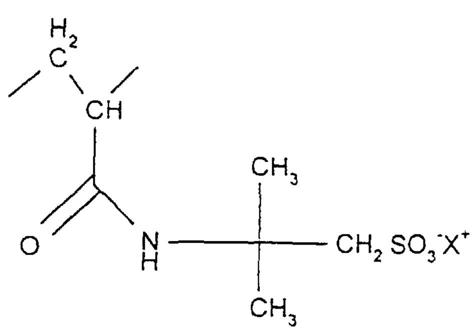

(III)

dans laquelle $X^+$ désigne un cation ou un mélange de cations.

b) de 0,01 à 10% en poids de motifs réticulants provenant d'au moins un monomère ayant au moins deux doubles liaisons oléfiniques ;

les proportions en poids étant définis par rapport au poids total du polymère.

Par ailleurs, selon un mode préféré de réalisation de l'invention, la phase huileuse de l'émulsion triple selon l'invention contient au moins une huile de silicone.

De façon avantageuse, cette émulsion peut être utilisée comme support d'actif sensible à l'eau et/ou à l'oxygène, notamment comme composition topique, en particulier cosmétique et/ou dermatologique.

Pour une application topique, l'émulsion selon l'invention doit contenir un milieu topiquement acceptable, c'est-à-dire compatible avec la peau, les muqueuses et les fibres kératiniques telles que les cheveux.

L'émulsion triple selon l'invention a l'avantage d'être stable tout en préservant l'activité des actifs. L'actif est de préférence introduit dans la phase aqueuse interne de l'émulsion triple, d'où il est libéré lors de l'application de la composition sur la peau, les muqueuses ou les cheveux.

Les copolymères émulsionnants utilisables dans l'émulsion selon la présente invention sont préparés en polymérisant une quantité prépondérante d'un monomère acide carboxylique monooléfiniquement insaturé ou de son anhydride, à une quantité plus faible de monomère ester acrylique à chaîne grasse. On entend par chaîne grasse un radical alkyle linéaire ou ramifiée, comportant de 8 à 30 atomes de carbone.

La quantité de monomère acide carboxylique ou de son anhydride va, de préférence, de 80 à 98 % en poids et plus particulièrement de 90 à 98 % en poids, tandis que le monomère ester acrylique est présent dans des quantités allant de 2 à 20 % en poids et plus particulièrement de 1 à 10 % en poids, les pourcentages étant calculés par rapport au poids des deux monomères.

Les monomères acides carboxyliques préférentiels sont choisis parmi ceux répondant à la formule (I) suivante :

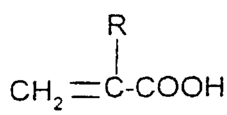
(I)

où R désigne l'hydrogène, un halogène, un groupe hydroxyle, un groupe lactone, un groupe lactame, un groupe cyanogène (-C=N), un groupe alkyle monovalent, un groupe aryle, un groupe alkylaryle, un groupe aralkyle ou un groupe cycloaliphatique.

Les monomères acides carboxyliques particulièrement préférés sont choisis parmi l'acide acrylique, l'acide méthacrylique ou leurs mélanges.

Les monomères esters acryliques à chaîne grasse sont préférentiellement choisis parmi ceux répondant à la formule (II) suivante :

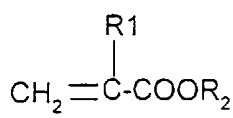
(II)

où $R_1$ est choisi dans le groupe formé par hydrogène, le radical méthyle et le radical éthyle, et $R_2$ est un radical alkyle en $C_8$-$C_{30}$.

Les monomères esters particulièrement préférés sont ceux pour lesquels $R_1$ est l'hydrogène ou un radical méthyle et $R_2$ est un radical alkyle en $C_{10}$-$C_{22}$.

Les copolymères émulsionnants peuvent être éventuellement réticulés à l'aide d'un agent réticulant utilisé en une quantité allant de 0,1 à 4 %, de préférence de 0,2 à 10 % en poids par rapport au poids total de monomères carboxyliques et de monomères esters acryliques. L'agent réticulant est choisi parmi les monomères polymérisables contenant un groupe $CH_2$=C- polymérisable et au moins un autre groupe polymérisable, dont les liaisons insaturées ne sont pas conjuguées l'un par rapport à l'autre.

Les copolymères émulsionnants de l'invention sont décrits dans la demande EP-A-0268164 et sont obtenus selon les méthodes de préparation décrites dans ce même document.

Les copolymères émulsionnants particulièrement préférés sont ceux présentant une viscosité mesurée au

viscosimètre BROOKFIELD dans une solution d'eau à 2% et à 25° C, inférieure ou égale à 5000 cPs (5 Pa.s) et plus préférentiellement de l'ordre d'environ 3000 cPs (3 Pa.s).

On utilise plus particulièrement un copolymère acrylate/$C_{10}$-$C_{30}$-alkylacrylate et notamment celui vendu sous le nom PEMULEN TR 1 par la Société GOODRICH.

Le copolymère émulsionnant est utilisé dans l'émulsion triple selon l'invention en une concentration allant, par exemple, de 0,05 à 3 % et de préférence de 0,1 à 1 %, et mieux de 0,2 à 0,8 % du poids total de l'émulsion.

Les poly-(acide 2-acrylamido 2-méthylpropane sulfonique) réticulés utilisés selon l'invention sont des polymères hydrosolubles ou gonflables dans l'eau.

De façon préférentielle, les polymères utilisés selon l'invention comportent un nombre de motifs de formule (III) dans une quantité suffisamment élevée pour obtenir un volume hydrodynamique du polymère en solution d'eau ayant un rayon allant de 10 à 500 nm, de distribution homogène et unimodale.

Les poly-(acide 2-acrylamido 2-méthylpropane sulfonique) réticulés plus particulièrement préférés comprennent de 98 à 99,5 % en poids de motifs de formule (III) et de 0,2 à 2 % en poids de motifs réticulants.

Dans la formule (III), $X^+$ représente un cation ou un mélange de cations choisis en particulier parmi un proton, un cation de métal alcalin, un cation équivalent de celui d'un métal alcalino-terreux ou l'ion ammonium.

De préférence, les poly-(acide 2-acrylamido 2-méthylpropane sulfonique) réticulés utilisés selon l'invention sont neutralisés à au moins 90 %, c'est-à-dire que au plus 10 % mole des cations $X^+$ dans la formule (III) sont des protons $H^+$.

Plus particulièrement, 90 à 100 % mole des cations sont des cations $NH_4^+$ et 0 à 10 % mole sont des protons $H^+$.

Les monomères de réticulation ayant au moins deux doubles liaisons oléfiniques sont choisis par exemple parmi le dipropylèneglycol-diallyléther, les polyglycol-diallyléthers, le triéthylèneglycol-divinyléther, l'hydroquinone-diallyl-éther, le tétrallyl-oxéthanoyle ou d'autres allyl ou vinyléthers alcools polyfonctionnels, le diacrylate de tétraéthylèneglycol, la triallylamine, le triméthylolpropane-diallyléther, le méthylen-bis-acrylamide ou le divinylbenzène.

Les monomères de réticulation ayant au moins deux doubles liaisons oléfiniques sont plus particulièrement choisis parmi ceux répondant à la formule générale (IV) suivante :

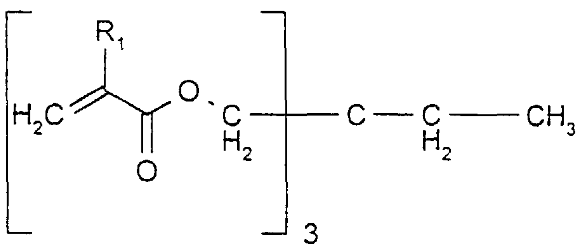
(IV)

dans laquelle $R_1$ désigne un atome d'hydrogène ou un radical alkyle en $C_1$-$C_4$ et plus particulièrement un radical méthyle. L'agent de réticulation préféré est le triméthylolpropane triacrylate.

La réaction de polymérisation des poly-(acide 2-acrylamido 2-méthylpropane sulfonique) de l'invention produit non seulement des chaînes linéaires mais aussi des molécules de polymère, ramifiées ou réticulées. Ces molécules peuvent être caractérisées notamment par leur comportement rhéologique dans l'eau mais plus particulièrement par la diffusion de la lumière dynamique.

Dans le cas de la caractérisation des molécules par la diffusion de la lumière dynamique, on mesure la distribution du volume hydrodynamique des structures du polymère. Les macromolécules dissoutes dans l'eau sont flexibles et entourées par une enveloppe de solvatation formée de molécules d'eau. Avec des polymères chargés comme ceux de l'invention, la taille des molécules dépend de la quantité de sel dans l'eau. Dans les solvants polaires, la charge uniforme le long de la chaîne principale du polymère conduit à une expansion importante de la chaîne polymérique. Le fait d'accroître la quantité de sel augmente la quantité d'électrolyte dans le solvant et écrante les charges uniformes du polymère. En plus des molécules transportées dans l'enveloppe de solvatation, les molécules de solvant sont fixées dans les cavités du polymère. Dans ce cas, les molécules de solvant font partie des macromolécules en solution et se déplacent à la même vitesse moyenne. Ainsi, le volume hydrodynamique décrit la dimension linéaire de la macromolécule et de ces molécules de solvatation.

Le volume hydrodynamique $v_h$ est déterminé par la formule suivante :

$$v_h = M/N_A \times (V_2 + dV_1)$$

avec :

M désignant la masse en grammes de la macromolécule non-dissoute ;
$N_A$ désignant le nombre d'Avogadro ;
$V_1$ désignant le volume spécifique du solvant ;
$V_2$ désignant le volume spécifique de la macromolécule ;
d la masse en grammes du solvant qui est associé avec 1 gramme de macromolécule non-dissoute.

Si la particule hydrodynamique est sphérique, il est alors facile de calculer à partir du volume hydrodynamique le rayon hydrodynamique par la formule :

$$v_h = 4\Pi R^3/3$$

avec R désignant le rayon hydrodynamique.

Les cas où les particules hydrodynamiques sont des sphères parfaites sont extrêmement rares. La majorité des polymères synthétiques impliquent des structures compactées ou des ellipsoïdes à haute excentricité. Dans ce cas, la détermination du rayon s'effectue sur une sphère qui est équivalente d'un point de vue frottement à la forme de la particule considérée.

En règle générale, on travaille sur des distributions de poids moléculaire et donc sur des distributions de rayon et de volume hydrodynamique. Pour les systèmes polydispersés, on doit calculer la distribution des coefficients de diffusion. De cette distribution, on en déduit les résultats relatifs à la distribution radiale et à la distribution des volumes hydrodynamiques.

Les volumes hydrodynamiques des polymères de l'invention sont en particulier déterminés par diffusion de la lumière dynamique à partir des coefficients de diffusion D selon STOKES-EINSTEIN de formule : $D = kT/6\Pi\eta R$ où k est la constante de Boltzmann, T la température absolue en degrés Kelvin, $\eta$ est la viscosité du solvant (eau) et R est le rayon hydrodynamique.

Ces coefficients de diffusion D sont mesurés selon la méthode de caractérisation d'un mélange de polymères par diffusion au LASER décrite dans les références suivantes :

(1) Pecora, R ; Dynamic Light Scattering ; Plenium Press, New York, 1976 ;
(2) Chu, B ; Dynamic Light Scattering ; Academic Press, New York, 1994 ;
(3) Schmitz, KS ; Introduction to Dynamic Light Scattering ; Academic Press, New York, 1990 ;
(4) Provincher S.W. ; Comp. Phys., 27, 213, 1982 ;
(5) Provincher S.W. ; Comp. Phys., 27, 229, 1982 ;
(6) ALV Laservertriebgesellschaft mbH, Robert Bosch Str. 47, D-63225 Langen, Germany ;
(7) ELS-Reinheimer Strasse 11, D-64846 Gross-Zimmern, Germany ;
(8) CHI WU et al, Macromolecules, 1995, 28,4914-4919.

Les poly-(acide 2-acrylamido 2-méthylpropane sulfonique) réticulés particulièrement préférés sont ceux présentant une viscosité mesurée au viscosimètre BROOKFIELD, mobile 4, à une vitesse de rotation de 100 tours/minutes, dans une solution d'eau à 2% et à 25 °C, supérieure ou égale à 1000 cPs (1 Pa.s) et plus préférentiellement allant de 5000 à 40000 cPs (5 Pa.s à 40 Pa.s) et plus particulièrement de 6500 à 35000 cPs (6,5 Pa.s à 35 Pa.s).

Les poly-(acide 2-acrylamido 2-méthylpropane sulfonique) réticulés de l'invention peuvent être obtenus selon le procédé de préparation comprenant les étapes suivantes :

(a) on disperse ou on dissout le monomère acide 2-acrylamido 2-méthylpropane sulfonique (AMPS) sous forme libre dans une solution de tertio-butanol ou d'eau et de tertio-butanol ;

(b) on neutralise la solution ou la dispersion de monomère AMPS obtenue en (a) par une ou plusieurs bases minérales ou organiques, de préférence l'ammoniaque $NH_3$ , dans une quantité permettant d'obtenir un taux de neutralisation des fonctions acides sulfoniques du polymère allant de 90 à 100 % ;

(c) on ajoute à la solution ou dispersion obtenue en (b) le ou les monomères réticulants ;

(d) on effectue une polymérisation radicalaire classique en la présence d'amorceurs de radicaux libres à une température allant de 10 à 150°C ; le polymère précipitant dans la solution ou la dispersion à base de tertio-butanol.

Le poly-(acide 2-acrylamido 2-méthylpropane sulfonique) réticulé est présent dans l'émulsion triple selon l'invention en une concentration allant, par exemple, de 0,01 à 10 %, de préférence de 0,5 à 2,5 % et mieux de 1,5 à 2,5 % du poids total de la composition. Selon une forme particulière de réalisation de l'invention, le poly-(acide 2-acrylamido 2-méthylpropane sulfonique) est incorporé dans l'émulsion triple en deux temps afin d'en faciliter la préparation.

Dans l'émulsion triple selon l'invention, l'émulsion primaire est une émulsion E/H dont la phase huileuse est, de façon avantageuse, essentiellement constituée par au moins un émulsionnant siliconé et /ou une huile de silicone.

Les émulsionnants siliconés utilisés dans l'émulsion triple de l'invention peuvent être choisis parmi les diméthicone copolyols et les alkyldiméthicone copolyols. On peut citer comme émulsionnant utilisable dans l'émulsion selon l'invention, le mélange de Polyglyceryl-4 isostearate/Cetyl dimethicone copolyol/Hexyl laurate vendu sous la dénomination « Abil WE 09 » par la société Goldschmidt, le Cetyl dimethicone copolyol vendu sous la dénomination « Abil EM 90 » par la société Goldschmidt et le mélange de cyclomethicone/dimethicone copolyol vendu sous la dénomination « Q2-3225C » par la société Dow Corning. La quantité d'émulsionnant siliconé dans l'émulsion selon l'invention va, par exemple, de 0,01 à 10 % et de préférence de 0,05 à 5 % du poids total de l'émulsion triple.

Les huiles de silicone peuvent être choisies par exemple parmi les silicones volatiles telles que le cyclopentadiméthylsiloxane et le cyclotétradiméthylsiloxane, les polydiméthylsiloxanes, les polyphényltriméthylsiloxanes, les silicones fluorées. La quantité d'huiles de silicone va de 0,5 à 40 % et de préférence de 2 à 30 % du poids total de l'émulsion triple.

L'émulsion primaire de l'invention peut en outre comprendre un ou plusieurs autres corps gras choisis parmi les cires, les gommes de silicone et les résines de silicone et éventuellement des huiles autres que les huiles de silicone, telles que les huiles d'origine végétale (huile d'abricot), les huiles de synthèse (isoparaffine hydrogénée). Ces corps gras peuvent être utilisés par exemple en une quantité allant de 0,05 à 10 % et de préférence de 0,5 à 5 % du poids total de l'émulsion triple.

Comme cires, on peut utiliser notamment les cires de silicone telles que les alcoxydiméthylsiloxanes, et plus particulièrement les stéaroxypolydiméthyl-siloxanes, les alkylpolysiloxanes et les polydiméthylsiloxanes à fonction mercapto.

Comme gommes, on peut utiliser notamment les gommes de silicone telles que les polydiméthylsiloxanes de haut poids moléculaire, les polydiméthylsiloxanes à terminaison hydroxyle (diméthiconols).

Comme résines, on peut utiliser notamment les résines de silicone telles que les triméthylsiloxysilicates.

Comme huiles autres que les huiles de silicone, on peut citer en particulier les huiles fluorées, les huiles d'origine animale ou végétale, les huiles minérales ou les huiles synthétiques.

L'émulsion primaire peut représenter par exemple de 10 à 40 % et de préférence de 20 à 30% du poids total de l'émulsion triple.

L'émulsion triple est préparée de manière classique par préparation de l'émulsion primaire et incorporation d'une quantité déterminée de l'émulsion primaire dans la phase aqueuse externe.

Selon un mode particulier de réalisation de l'invention, l'émulsion primaire contient une partie de l'huile de silicone, par exemple au maximum 20 % en poids par rapport au poids total de l'émulsion primaire, et on ajoute ensuite le reste d'huile de silicone à la quantité d'émulsion primaire utilisée pour préparer l'émulsion triple, avant d'ajouter le mélange dans la phase aqueuse externe.

Comme indiqué en début de description, l'un des intérêts majeurs de l'émulsion conforme à l'invention est que cette dernière peut contenir, tout en présentant un caractère stable, des principes actifs, tant cosmétiques que thérapeutiques, notamment les actifs sensibles à l'eau et/ou à l'oxygène et aussi les actifs à caractère acide, ces actifs pouvant donc être notamment choisis parmi tous ceux habituellement utilisés à ce jour dans le domaine de la cosmétique, de la dermatologie ou du médicament.

Comme actifs sensibles à l'eau et/ou à l'oxygène, on peut citer notamment les enzymes (par exemple lactoperoxydase, lipase, protéase, phospholipase, cellulases), les extraits naturels tels que thé vert, extrait de mélisse, extrait de thym, les oligomères procyannidoliques (OPC) tels que l'OPC d'aubépine, l'OPC de pin et l'OPC de raisin, les vitamines et notamment l'acide ascorbique (vitamine C) et ses esters, le rétinol (vitamine A) et ses esters, les dérivés phosphatés et glucosylés, l'urée et la rutine.

Outre l'acide ascorbique cité ci-dessus, on peut citer aussi, entre autres, comme actifs à caractère acide, l'acide kojique, l'acide caféique, les bêta-hydroxyacides tels que l'acide salicylique et ses dérivés, les alpha-hydroxyacides et les alpha-céto-acides tels que l'acide lactique, l'acide méthyllactique, l'acide citrique, l'acide mandélique, l'acide glucuronique, l'acide glycolique, l'acide pyruvique, l'acide 2-hydroxybutanoique, l'acide 2-hydroxypentanoique, l'acide 2-hydroxyhexanoique, l'acide 2-hydroxyheptanoique, l'acide 2-hydroxyoctanoique, l'acide 2-hydroxynonanoique, l'acide 2-hydroxydécanoique, l'acide 2-hydroxyundécanoique, l'acide 2-hydroxydodécanoique, l'acide 2-hydroxytetradécanoique, l'acide 2-hydroxyhexadecanoique, l'acide 2-hydroxyoctadécanoique, l'acide 2-hydroxytetraécosanoique, l'acide 2-hydroxyeicosanoique, l'acide benzylique, l'acide phényllactique, l'acide gluconique, l'acide galacturonique, l'acide aleuritique, l'acide ribonique, l'acide tartronique, l'acide tartrique, l'acide malique, l'acide fumarique, l'acide rétinoique et ses dérivés, l'acide benzène 1,4-di-(3-méthylidène 10-camphosulfonique) et leurs mélanges. Il peut s'agir

également de tous les composés naturels ou synthétiques contenant de tels acides, en particulier les extraits végétaux, et plus spécialement les extraits de fruits.

Selon un mode particulier de réalisation de l'invention, l'émulsion triple de l'invention contient comme actifs de l'acide ascorbique et du rétinol, seuls ou en mélange.

L'actif ou les actifs peuvent être par exemple présents en une concentration allant de 0,01 à 20 %, de préférence de 0,1 à 10 % et mieux de 0,5 à 5 % du poids total de l'émulsion.

Selon un mode particulier de réalisation de l'invention, l'émulsion primaire contient un ou plusieurs polyols en une quantité suffisante pour abaisser l'activité en eau de la phase interne de l'émulsion triple, afin de stabiliser les actifs sensibles à l'eau tels que l'acide ascorbique. De manière avantageuse, l'activité en eau de l'émulsion primaire va de 0,5 à 0,8. Les polyols peuvent, par exemple, être choisis parmi la glycérine, les glycols tels que le propylène glycol et le PEG 8 et les silicones comportant des groupes hydroxyle. Les polyols peuvent être présents en une quantité allant, de préférence, de 0,5 à 50 % et préférentiellement de 25 à 45 % du poids total de l'émulsion primaire.

Comme indiqué précédemment, les émulsions E/H/E selon l'invention peuvent être utilisées dans différentes applications topiques, notamment cosmétiques et/ou dermatologiques. La composition à base de cette émulsion peut constituer notamment des compositions de nettoyage, de protection, de traitement ou de soin de la peau et/ou des cheveux, en particulier pour le visage, pour le cou, pour les mains, pour les cheveux, pour le cuir chevelu ou pour le corps, ainsi que pour les cils.

Aussi, l'invention a encore pour objet l'utilisation cosmétique de la composition selon l'invention pour nettoyer et/ou traiter et/ou protéger la peau et/ou les muqueuses et/ou les fibres kératiniques, c'est-à-dire les cheveux et/ou les cils.

L'invention a aussi pour objet l'utilisation de la composition selon l'invention pour la préparation d'une composition dermatologique destinée à nettoyer et/ou traiter et/ou protéger la peau et/ou les muqueuses et/ou les fibres kératiniques, c'est-à-dire les cheveux et/ou les cils.

L'invention a aussi pour objet un procédé cosmétique et/ou dermatologique pour nettoyer et/ou traiter et/ou protéger la peau, les muqueuses et/ou les fibres kératiniques, caractérisé en ce qu'il consiste à appliquer sur la peau et/ou les muqueuses et/ou les fibres kératiniques, une composition telle que définie ci-dessus.

La composition selon l'invention peut constituer notamment des crèmes de protection, de traitement ou de soin pour le visage, pour les mains, pour les pieds, des laits corporels de protection ou de soin, des lotions, gels ou mousses pour le soin de la peau, des muqueuses, des cheveux et du cuir chevelu.

De façon connue, la composition de l'invention peut contenir également des adjuvants habituels dans les domaines cosmétique et dermatologique, tels que les tensioactifs, notamment les tensioactifs moussants, les actifs hydrophiles ou lipophiles autres que les actifs indiqués ci-dessus, les conservateurs, les antioxydants, les séquestrants, les solvants, les parfums, les charges, les filtres, les absorbeurs d'odeur et les matières colorantes. Les quantités de ces différents adjuvants sont celles classiquement utilisées dans les domaines considérés, et par exemple de 0,01 % à 15 % du poids total de la composition. Elle peut contenir également des vésicules lipidiques formées de lipides ioniques ou non ioniques.

Ces adjuvants, selon leur nature, peuvent être introduits dans la phase grasse ou dans la phase aqueuse.

Comme actifs hydrophiles, on peut utiliser par exemple, outre les polyols indiqués ci-dessus, les protéines ou les hydrolysats de protéine, les acides aminés, l'allantoïne, les sucres et les dérivés de sucre, l'amidon.

Comme actifs lipophiles, on peut utiliser par exemple le tocophérol (vitamine E) et ses dérivés, les acides gras essentiels, les céramides, les huiles essentielles.

Les exemples ci-après de compositions selon l'invention sont donnés à titre d'illustration et sans caractère limitatif. Les quantités y sont données en % en poids, sauf mention contraire.

<u>Exemple de préparation d'un poly(acide 2-acrylamido 2-méthylpropane sulfonique} réticulé et neutralisé par de l'ammoniaque</u>

Dans un ballon de 5 litres muni d'un agitateur, d'un réfrigérant à reflux, d'un thermomètre et d'un dispositif de conduite pour l'azote et pour l'ammoniaque, on introduit 2006,2 g de tertio-butanol puis 340,0 g d'acide 2-acrylamido 2-méthylpropane sulfonique que l'on disperse dans la solution sous forte agitation. Après 30 minutes, on ajoute l'ammoniaque par le conduit supérieur du ballon et on maintient le mélange réactionnel pendant 30 minutes à température ambiante jusqu'à l'obtention d'un pH de l'ordre de 6-6,5. On introduit ensuite 32,0 g d'une solution de triméthylolpropane triacrylate à 25% dans le tertio-butanol et on chauffe jusqu'à 60°C tandis que le milieu réactionnel est simultanément rendu inerte par apport d'azote dans le ballon. Une fois cette température atteinte, on ajoute du dilauroylperoxyde. La réaction se déclenche aussitôt, ce qui se traduit par une montée de température et par une précipitation du polymérisat. 15 minutes après le début de la polymérisation, on introduit un courant d'azote. 30 minutes après l'ajout de l'amorceur, la température du milieu réactionnel atteint un maximum de 65-70°C. 30 minutes après avoir atteint cette température, on chauffe au reflux et on maintient dans ces conditions pendant 2 heures. On observe au cours de la réaction la

formation d'une pâte épaisse. On refroidit jusqu'à la température ambiante et on filtre le produit obtenu. La pâte récupérée est ensuite séchée sous vide à 60-70°C pendant 24 heures. On obtient 391 g de poly-(acide 2-acrylamido 2-méthylpropane sulfonique) réticulé et neutralisé, ayant viscosité mesurée au viscosimètre BROOKFIELD, mobile 4, à une vitesse de rotation de 100 tours/minute dans une solution d'eau à 2 % et à 25 °C allant de 15000 cPs à 35000 cPs (15 Pa.s à 35 Pa.s). La viscosité du polymère sera choisie et contrôlée selon des moyens classiques en fonction de l'application cosmétique envisagée.

Le rayon hydrodynamique du polymère obtenu dans une solution aqueuse déterminé par diffusion de la lumière dynamique est de 440 nm.

Exemple 1 : Emulsion hydratante pour l'éclat du teint

1. Emulsion primaire :

*Phase A*:

- Abil WE 09 2,5 %
- Huile de silicone volatile 17,5 %
- Polydiméthylsiloxane 4 %

*Phase B* :

- Glycérine 39 %
- Acide ascorbique 15 %
- Séquestrant 0,1 %
- conservateur 0,8 %
- Eau déminéralisée 21,1 %

2. Emulsion triple:

*Phase A :*

- Emulsion primaire 20 %
- Huile de silicone 10 %

*Phase B:*

- Poly-(acide 2-acrylamido 2-méthylpropane sulfonique) réticulé et neutralisé par de l'ammoniaque, préparé selon le procédé de l'exemple de préparation (viscosité de l'ordre de 16 Pa.s dans une solution d'eau à 2% et à 25°C) 0,5 %
- Copolymère acrylate/$C_{10}$-$C_{30}$-alkylacrylate (Pemulen TR1) 0,3 %
- Conservateurs 1 %
- Eau déminéralisée 40 %

*Phase C*:

- triéthanolamine 0,3 %
- Eau déminéralisée 2 %

*Phase D* :

- Poly-(acide 2-acrylamido 2-méthylpropane sulfonique) réticulé et neutralisé par de l'ammoniaque, préparé selon le procédé de l'exemple de préparation (viscosité de l'ordre de 16 Pa.s, dans une solution d'eau à 2% et à 25°C) 1,5 %
- Eau déminéralisée 24,4 %

L'émulsion triple est préparée de la manière suivante :

1. On prépare l'émulsion primaire en mélangeant les constituants de la phase A à température ambiante, en mélangeant par ailleurs les constituants de la phase B à température ambiante et en versant lentement la phase B dans la phase A sous agitation rapide.
2. Pour préparer l'émulsion triple, on prépare les différentes phases, puis on verse lentement la phase A dans la phase B sous agitation rapide. On y ajoute la phase C puis la phase D. On agite jusqu'à homogénéisation complète.

Exemple 2 : on peut préparer une composition analogue à celle de l'exemple 1 en remplaçant l'acide ascorbique par l'acide glycolique.

Exemple 3 : Emulsion pour le lissage de la peau

1. Emulsion primaire :

*Phase A :*

- Abil WE09 2,5 %
- Huile de silicone volatile 17.5 %
- Polydiméthylsiloxane 4 %

*Phase B :*

- Glycérine 45 %
- Acide ascorbique 10 %
- Conservateur 0,8 %
- Eau déminéralisée 20,2 %

2. Emulsion triple :

*Phase A :*

- Emulsion primaire 20 %
- Huile de silicone volatile 10 %

*Phase B* :

- Poly-(acide 2-acrylamido 2-méthylpropane sulfonique) réticulé et neutralisé par de l'ammoniaque, préparé selon le procédé de l'exemple de préparation (viscosité de l'ordre de 16 Pa.s dans une solution d'eau à 2% et à 25°C) 0,5 %
- Copolymère acrylate/$C_{10}$-$C_{30}$-alkylacrylate (Pemulen TR1) 0,3 %

- Conservateurs 1 %
- Eau déminéralisée 40 %

*Phase C :*

- triéthanolamine 0,3 %
- Eau déminéralisée 2 %

*Phase D :*

- Poly-(acide 2-acrylamido 2-méthylpropane sulfonique) réticulé et neutralisé par de l'ammoniaque, préparé selon le procédé de l'exemple de préparation (viscosité de l'ordre de 16 Pa.s dans une solution d'eau à 2% et à 25°C) 1,5 %
- Eau déminéralisée 24,4 %

L'émulsion triple est préparée selon le même mode opératoire que pour l'exemple 1.
On obtient une crème blanche, apte à lisser la peau.

Exemple 4 : Emulsion de soin

1. Emulsion primaire :

*Phase A :*

- Abil WE 09 3,5 %
- Isoparaffine hydrogénée 23 %

*Phase B :*

- Glycérine 39 %
- conservateur 1 %
- Eau déminéralisée 33,7 %

2. Emulsion triple:

*Phase A :*

- Emulsion primaire 20 %
- Isoparaffine hydrogénée 4 %
- Huile d'abricot 5 %
- Rétinol 0,1 %
- BHT (antioxydant) 0,11 %

*Phase B* :

- Poly-(acide 2-acrylamido 2-méthylpropane sulfonique) réticulé et neutralisé par de l'ammoniaque, préparé selon

le procédé de l'exemple de préparation (viscosité de l'ordre de 16 Pa.s dans une solution d'eau à 2% et à 25°C) 1 %
- Copolymère acrylate/$C_{10}$-$C_{30}$-alkylacrylate (Pemulen TR1) 0,6 %
- conservateur 1 %
- Eau déminéralisée 64,09 %

*Phase C* :

- Triéthanolamine 1,1 %
- Eau déminéralisée 3 %

L'émulsion triple est préparée selon le même mode opératoire que pour l'exemple 1. On obtient une crème blanche apte à lisser la peau.

Exemple 5 : Emulsion de soin

1. Emulsion primaire :

*Phase A :*

- Abil WE 09 3,5 %
- Isoparaffine hydrogénée 20,5 %

*Phase B :*

- Glycérine 39 %
- conservateur 0,8 %
- Acide ascorbique 15 %
- Eau déminéralisée 21,2 %

2. Emulsion triple :

*Phase A :*

- Emulsion primaire 20 %
- Isoparaffine hydrogénée 4 %
- Huile d'abricot 5 %
- Rétinol 0,1 %
- Lipocarnosine 0,1 %

*Phase B :*

- Poly-(acide 2-acrylamido 2-méthylpropane sulfonique) réticulé et neutralisé par de l'ammoniaque, préparé selon le procédé de l'exemple de préparation (viscosité de l'ordre de 16 Pa.s dans une solution d'eau à 2% et à 25°C) 1 %
- Copolymère acrylate/$C_{10}$-$C_{30}$-alkylacrylate (Pemulen TR1) 0,6 %
- conservateur 1 %
- Eau déminéralisée 64,1 %

*Phase C* :

- Triéthanolamine 1,1 %
- Eau déminéralisée 3 %

L'émulsion triple est préparée selon le même mode opératoire que pour l'exemple 1. On obtient une crème blanche apte à lisser la peau et à améliorer l'éclat du teint.

Test de stabilité : le test suivant est destiné à mettre en évidence la supériorité de l'association de gélifiants revendiquée par rapport à d'autres associations. L'exemple selon l'invention est l'exemple 1. Dans l'exemple comparatif, le poly-(acide 2-acrylamido 2-méthylpropane sulfonique) réticulé est remplacé par du Carbopol 980. Le tableau suivant donne les caractéristiques de l'émulsion triple obtenue :

| Polymère | Exemple 1 selon l'invention | Exemple comparatif |
|---|---|---|
| Poly-(acide 2-acrylamido 2-méthylpropane sulfonique) de l'exemple de préparation | X | |
| Copolymère acrylate/$C_{10}$-$C_{30}$-alkylacrylate (Pemulen TR1) | X | |
| Copolymère acrylate/$C_{10}$-$C_{30}$-alkylacrylate (Carbopol 1382) | | X |
| Carbopol 980 (1) | | X |
| Formation d'une émulsion triple | Oui | Oui |
| Stabilité après 24 heures | Stable | Instable |

(1) Carbopol 980 : polymère carboxyvinylique

Ce tableau montre que seule l'association selon l'invention permet d'obtenir une émulsion triple stable en présence de composé acide.

## Revendications

**1.** Emulsion triple eau/huile/eau comportant une phase aqueuse externe gélifiée, une phase huileuse constituant avec une phase aqueuse interne une émulsion primaire eau/huile, caractérisée en ce que la phase huileuse comprend un émulsionnant choisi parmi les alkyldiméthicone copolyols et les diméthicone copolyols et en ce que la phase aqueuse externe contient :

1) au moins un copolymère émulsionnant constitué d'une fraction majoritaire d'un monomère acide carboxylique monooléfiniquement insaturé en $C_3$-$C_6$ ou de son anhydride et d'une fraction minoritaire de monomère ester gras d'acide acrylique, et

2) au moins un poly-(acide acrylamidométhyl propane sulfonique) réticulé comprenant, distribués de façon aléatoire :

a) de 90 à 99,9% en poids de motifs de formule générale (III) suivante :

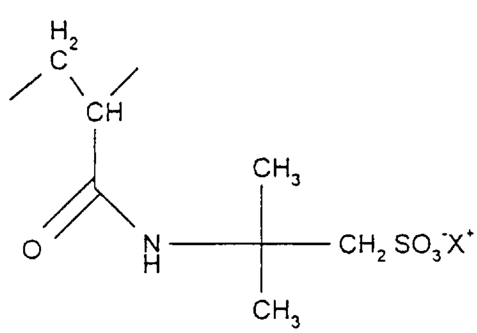

(III)

dans laquelle $X^+$ désigne un cation ou un mélange de cations.

b) de 0,01 à 10% en poids de motifs réticulants provenant d'au moins un monomère ayant au moins deux doubles liaisons oléfiniques ;

les proportions en poids étant définis par rapport au poids total du polymère.

**2.** Emulsion selon la revendication 1, caractérisée par le fait que la quantité de monomère acide carboxylique ou de son anhydride dans le copolymère émulsionnant varie de 80 à 98% en poids et que la quantité de monomère ester varie de 20 à 2% en poids, les pourcentages en poids étant exprimés par rapport au poids total des deux monoméres.

**3.** Emulsion selon la revendication 1 ou 2, caractérisée par le fait que le monomère acide carboxylique est un composé de formule (I) :

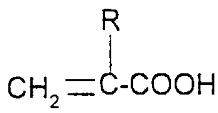

(I)

où R désigne l'hydrogène, un halogène, un groupe hydroxyle, un groupe lactone, un groupe lactame, un groupe cyanogène, un groupe alkyle monovalent, un groupe aryle, un groupe alkylaryle, un groupe aralkyle ou un groupe cycloaliphatique et que le monomère ester est un composé de formule (II) :

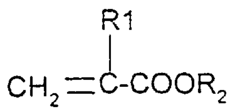

(II)

où $R_1$ est choisi dans le groupe formé par l'hydrogène, un radical méthyle et un radical éthyle et $R_2$ est un groupe alkyle en $C_8$-$C_{30}$.

**4.** Emulsion selon la revendication 3, caractérisée par le fait que le monomère acide carboxylique est choisi parmi l'acide acrylique, l'acide méthacrylique ou leurs mélanges, et que le monomère ester est choisi parmi les monomères de formule (II) dans laquelle $R_1$ est l'hydrogène ou le radical méthyle et $R_2$ est un groupe alkyle en $C_{10}$-$C_{22}$.

**5.** Emulsion selon l'une quelconque des revendications précédentes, caractérisée par le fait que le copolymère émulsionnant est présent en une quantité allant de 0,05 à 3 % et de préférence de 0,1 à 1 % du poids total de l'émulsion.

**6.** Emulsion selon l'une quelconque des revendications précédentes, caractérisée par le fait que le poly-(acide 2-acrylamido 2-méthylpropane sulfonique) réticulé comporte de 98 à 99,5 % en poids de motifs de formule (III) et de 0,2 à 2 % en poids de motifs réticulants.

**7.** Emulsion selon l'une quelconque des revendications précédentes, caractérisée par le fait que le poly-(acide 2-acrylamido 2-méthylpropane sulfonique) réticulé est neutralisé à au moins 90 %.

**8.** Emulsion selon l'une quelconque des revendications précédentes, caractérisée par le fait que dans la formule (III) le cation $X^+$ est $NH_4^+$.

**9.** Emulsion selon l'une quelconque des revendications précédentes, caractérisée par le fait que les monomères réticulants répondent à la formule générale (IV) suivante :

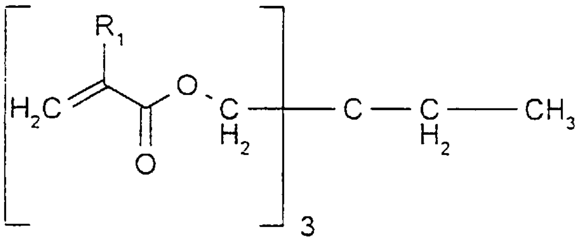 (IV)

dans laquelle $R_1$ désigne un atome d'hydrogène ou un alkyle en $C_1$-$C_4$.

**10.** Emulsion selon l'une quelconque des revendications précédentes, caractérisée par le fait que le poly-(acide 2-acrylamido 2-méthylpropane sulfonique) est réticulé par le triméthylol propane triacrylate.

**11.** Emulsion selon l'une quelconque des revendications précédentes, caractérisée par le fait que le poly-(acide 2-acrylamido 2-méthylpropane sulfonique) réticulé présente une viscosité mesurée au viscosimètre BROOKFIELD, mobile 4, à une vitesse de rotation de 100 tours/minutes, dans une solution d'eau à 2% et à 25 °C, supérieure ou égale à 1 Pa.s, de préférence allant de 5 à 40 Pa.s.

**12.** Emulsion selon l'une quelconque des revendications précédentes, caractérisée par le fait que le poly-(acide 2-acrylamido 2-méthylpropane sulfonique) réticulé est présent en une concentration allant de 0,01 à 10 %, de préférence de 0,5 à 2,5 % du poids total de la composition.

**13.** Emulsion selon l'une quelconque des revendications précédentes, caractérisée en ce que la phase huileuse comprend au moins une huile de silicone en une quantité allant de 0,5 à 40 % du poids total de l'émulsion.

**14.** Emulsion selon la revendication précédente, caractérisée en ce que l'huile de silicone est choisie dans le groupe comprenant les silicones volatiles, les polydiméthylsiloxanes, les polyphényltriméthylsiloxanes, les silicones fluorées.

**15.** Emulsion selon l'une quelconque des revendications précédentes, caractérisée en ce que l'émulsionnant est présent en une quantité allant de 0,01 à 10 % et de préférence de 0,05 à 5 % du poids total de l'émulsion.

**16.** Emulsion selon l'une quelconque des revendications précédentes, caractérisée en ce que la phase huileuse contient en outre un ou plusieurs autres corps gras choisis parmi les cires, les gommes de silicone, les résines de silicone, les huiles fluorées, les huiles d'origine animale, les huiles d'origine végétale, les huiles minérales et les huiles synthétiques.

**17.** Emulsion selon l'une quelconque des revendications précédentes, caractérisée en ce que l'émulsion primaire eau/huile représente de 10 à 40 % et de préférence de 20 à 30 % du poids total de l'émulsion.

**18.** Emulsion selon l'une quelconque des revendications précédentes, caractérisée en ce que l'émulsion primaire eau/huile contient au moins un polyol.

**19.** Emulsion selon la revendication précédente, caractérisée en ce que le ou les polyols sont présents en une quantité allant de 0,5 à 50 % et de préférence de 25 à 45 % du poids de l'émulsion primaire.

**20.** Composition topique, caractérisée en ce qu'elle contient une émulsion selon l'une quelconque des revendications précédentes et au moins un actif.

**21.** Composition selon la revendication précédente, caractérisée en ce que l'actif est choisi dans le groupe comprenant les actifs sensibles à l'eau et/ou à l'oxygène et les actifs acides.

**22.** Composition selon la revendication 20 ou 21, caractérisée en ce que l'actif est choisi parmi les enzymes, les extraits naturels, les oligomères procyannidoliques, les vitamines, les dérivés phosphatés et glucosylés, l'urée, la rutine, l'acide kojique, l'acide caféique, les bêta-hydroxyacides, les alpha-hydroxyacides, l'acide rétinoique et ses dérivés, l'acide benzène 1,4-di-(3-méthylidène 10-camphosulfonique), les extraits végétaux contenant un tel acide,

et leurs mélanges.

**23.** Composition selon l'une quelconque des revendications 20 à 22, caractérisée en ce que l'actif est choisi parmi l'acide ascorbique, le rétinol, l'acide salicylique, l'acide lactique, l'acide méthyllactique, l'acide citrique, l'acide mandélique, l'acide glucuronique, l'acide glycolique, l'acide pyruvique, l'acide 2-hydroxybutanoique, l'acide 2-hydroxypentanoique, l'acide 2-hydroxyhexanoique, l'acide 2-hydroxyheptanoique, l'acide 2-hydroxyoctanoique, l'acide 2-hydroxynonanoique, l'acide 2-hydroxydécanoique, l'acide 2-hydroxyundécanoique, l'acide 2-hydroxydodécanoique, l'acide 2-hydroxytetradécanoique, l'acide 2-hydroxyhexadecanoique, l'acide 2-hydroxyoctadécanoique, l'acide 2-hydroxytetraécosanoique, l'acide 2-hydroxyeicosanoique, l'acide benzylique, l'acide phényllactique, l'acide gluconique, l'acide galacturonique, l'acide aleuritique, l'acide ribonique, l'acide tartronique, l'acide tartrique, l'acide malique, l'acide fumarique, leurs dérivés et leurs mélanges.

**24.** Composition selon l'une quelconque des revendications 20 à 23, caractérisée en ce qu'elle contient comme actifs de l'acide ascorbique et du rétinol.

**25.** Composition selon l'une quelconque des revendications 20 à 24, caractérisée en ce que l'actif est présent en une concentration allant de 0,01 à 20 % et de préférence de 0,1 à 10 % du poids total de la composition.

**26.** Composition selon l'une quelconque des revendications 20 à 25, caractérisée en ce qu'elle comprend au moins un adjuvant lipophile ou hydrophile choisi parmi les conservateurs, les antioxydants, les séquestrants, les solvants, les parfums, les charges, les filtres, les absorbeurs d'odeur, les matières colorantes, les actifs hydrophiles ou lipophiles, et les vésicules lipidiques.

**27.** Utilisation cosmétique de la composition selon l'une quelconque des revendications 20 à 26 pour nettoyer et/ou traiter et/ou protéger la peau et/ou les muqueuses et/ou les fibres kératiniques.

**28.** Utilisation de la composition selon l'une quelconque des revendications 20 à 26 pour la préparation d'une composition dermatologique destinée à nettoyer et/ou traiter et/ou protéger la peau et/ou les muqueuses et/ou les fibres kératiniques.

**29.** Procédé cosmétique pour nettoyer et/ou traiter et/ou protéger la peau et/ou les fibres kératiniques, caractérisé en ce qu'il consiste à appliquer sur la peau, les muqueuses et/ou les fibres kératiniques, une composition selon l'une quelconque des revendications 20 à 26.

## Patentansprüche

**1.** Dreifache Wasser/Öl/Wasser-Emulsion, die eine äußere wäßrige Phase in Gelform und eine ölphase aufweist, welche mit einer inneren wäßrigen Phase eine primäre Wasser-in-Öl-Emulsion bildet, dadurch gekennzeichnet, daß die Ölphase einen Emulgator enthält, der unter den Alkyldimethiconcopolyolen und den Dimethiconcopolyolen ausgewählt ist, und dadurch, daß die äußere wäßrige Phase enthält:

1) mindestens ein emulgierendes Copolymer, das aus monoolefinisch ungesättigten $C_{3-6}$-Carbonsäuremonomeren oder ihren Anhydriden als Hauptanteil und in geringerem Anteil Acrylfettestermonomeren besteht, und
2) mindestens eine vernetzte Poly(acrylamido-methylpropansulfonsäure), die zufällig verteilt enthält:

a) 90 bis 99,9 Gew.-% Einheiten der folgenden augemeinen Formel (III)

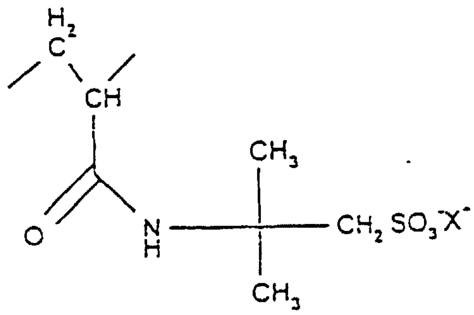

(III),

worin $X^+$ ein Kation oder ein Gemisch von Kationen bedeutet; und

b) 0,01 bis 10 Gew.-% vernetzende Einheiten, die von mindestens einem Monomer stammen, das mindestens zwei olefinische Doppelbindungen aufweist;

wobei die Mengenanteile in Gewichtsprozent bezogen auf das Gesamtgewicht des Polymers angegeben sind.

**2.** Emulsion nach Anspruch 1, dadurch gekennzeichnet, daß der Mengenanteil des Carbonsäuremonomers oder seines Anhydrids in dem emulgierenden Polymer im Bereich von 80 bis 98 Gew.-% und der Mengenanteil des Estermonomers im Bereich von 2 bis 20 Gew.-% liegt, wobei die Mengenanteile bezogen auf das Gesamtgewicht der beiden Monomere angegeben sind.

**3.** Emulsion nach Anspruch 1 oder 2, dadurch gekennzeichnet, daß das Carbonsäuremonomer eine Verbindung der folgenden Formel (I) ist:

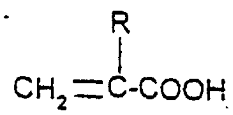

(I),

worin R Wasserstoff, ein Halogen, eine Hydroxygruppe, eine Lactongruppe, eine Lactamgruppe, eine Cyanogengruppe, eine einwertige Alkylgruppe, eine Arylgruppe, eine Alkylarylgruppe, eine Aralkylgruppe oder eine cycloaliphatische Gruppe bedeutet, und dadurch, daß das Estermonomer eine Verbindung der folgenden Formel (II) ist:

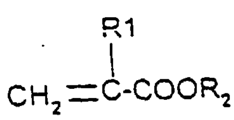

(II),

worin $R_1$ unter Wasserstoff, Methyl und Ethyl ausgewählt ist und $R_2$ eine $C_{8-30}$-Alkylgruppe bedeutet.

**4.** Emulsion nach Anspruch 3, dadurch gekennzeichnet, daß das Carbonsäuremonomer unter Acrylsäure, Methacrylsäure oder deren Gemischen, und das Estermonomer unter den Monomeren der Formel (II), worin $R_1$ Wasserstoff oder Methyl und $R_2$ eine $C_{10-22}$-Alkylgruppe bedeutet, ausgewählt ist.

**5.** Emulsion nach einem der vorhergehenden Ansprüche, dadurch gekennzeichnet, daß das emulgierende Copolymer in einem Mengenanteil von 0,05 bis 3 % und vorzugsweise von 0,1 bis 1 % des Gesamtgewichts der Emulsion vorliegt.

**6.** Emulsion nach einem der vorhergehenden Ansprüche, dadurch gekennzeichnet, daß die vernetzte Poly(2-acrylamido-2-methyl-propansulfonsäure) 98 bis 99,5 Gew.-% Einheiten der Formel (III) und 0,2 bis 2 Gew.-% Vernetzungseinheiten enthält.

**7.** Emulsion nach einem der vorhergehenden Ansprüche, dadurch gekennzeichnet, daß die vernetzte Poly(2-acrylamido-2-methyl-propansulfonsäure) zu mindestens 90 % neutralisiert ist.

**8.** Emulsion nach einem der vorhergehenden Ansprüche, dadurch gekennzeichnet, daß in der Formel (III) das Kation $X^+$ $NH_4^+$ bedeutet.

**9.** Emulsion nach einem der vorhergehenden Ansprüche, dadurch gekennzeichnet, daß die Monomere zur Vernetzung der folgenden allgemeinen Formel (IV) entsprechen:

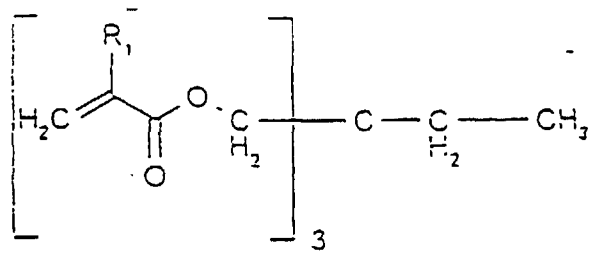

(IV),

worin $R_1$ Wasserstoff oder $C_{1-4}$-Alkyl bedeutet.

**10.** Emulsion nach einem der vorhergehenden Ansprüche, dadurch gekennzeichnet, daß die Poly(2-acrylamido-2-methyl-propansulfonsäure) mit Trimethylolpropantriacrylat vernetzt ist.

**11.** Emulsion nach einem der vorhergehenden Ansprüche, dadurch gekennzeichnet, daß die vernetzte Poly(2-acrylamido-2-methyl-propansulfonsäure) eine mit einem BROOKFIELD-Viskosimeter (bewegliches Teil 4) bei einer Rotationsgeschwindigkeit von 100 U/min in einer 2%igen wäßrigen Lösung bei 25 °C bestimmte Viskosität von mindestens 1 Pa·s und vorzugsweise im Bereich von 5 bis 40 Pa·s aufweist.

**12.** Emulsion nach einem der vorhergehenden Ansprüche, dadurch gekennzeichnet, daß die vernetzte Poly(2-acrylamido-2-methyl-propansulfonsäure) in einer Konzentration im Bereich von 0,01 bis 10 % und vorzugsweise von 0,5 bis 2,5 % des Gesamtgewichts der Zusammensetzung vorliegt.

**13.** Emulsion nach einem der vorhergehenden Ansprüche, dadurch gekennzeichnet, daß die ölphase mindestes ein Siliconöl in einem Mengenanteil von 0,5 bis 40 % des Gesamtgewichts der Emulsion enthält.

**14.** Emulsion nach dem vorhergehenden Anspruch, dadurch gekennzeichnet, daß das Siliconöl unter den flüchtigen Siliconen, Polydimethylsiloxanen, Polyphenyltrimethylsiloxanen und fluorierten Siliconen ausgewählt ist.

**15.** Emulsion nach einem der vorhergehenden Ansprüche, dadurch gekennzeichnet, daß der Emulgator in einem Mengenanteil im Bereich von 0,01 bis 10 % und vorzugsweise von 0,05 bis 5 % des Gesamtgewichts der Emulsion vorliegt.

**16.** Emulsion nach einem der vorhergehenden Ansprüche, dadurch gekennzeichnet, daß die Ölphase ferner eine oder mehrere Fettsubstanzen enthält, die unter den Wachsen, Silicongummis, Siliconharzen, fluorierten Ölen, Ölen tierischen Ursprungs, Ölen pflanzlichen Ursprungs, Mineralölen und synthetischen Ölen ausgewählt sind.

**17.** Emulsion nach einem der vorhergehenden Ansprüche, dadurch gekennzeichnet, daß die primäre Wasser-in-Öl-Emulsion 10 bis 40 % und vorzugsweise 20 bis 30 % des Gesamtgewichts der Emulsion ausmacht.

**18.** Emulsion nach einem der vorhergehenden Ansprüche, dadurch gekennzeichnet, daß die primäre Wasser-in-Öl-Emulsion mindestens ein Polyol enthält.

**19.** Emulsion nach dem vorhergehenden Ansprüche, dadurch gekennzeichnet, daß das Polyol oder die Polyole in einem Mengenanteil von 0,5 bis 50 % und vorzugsweise von 25 bis 45 % des Gesamtgewichts der primären Emulsion vorliegen.

**20.** Topische Zusammensetzung, dadurch gekennzeichnet, daß sie eine Emulsion nach einem der vorhergehenden Ansprüche und einen Wirkstoff enthält.

**21.** Zusammensetzung nach dem vorhergehenden Anspruch, dadurch gekennzeichnet, daß der Wirkstoff unter den gegenüber Wasser und/oder Sauerstoff empfindlichen Wirkstoffen und den sauren Wirkstoffen ausgewählt ist.

**22.** Zusammensetzung nach Anspruch 20 oder 21, dadurch gekennzeichnet, daß der Wirkstoff unter den Enzymen, natürlichen Extrakten, Procyannidol-Oligomeren, Vitaminen, phosphatierten und glucosylierten Derivaten, Harnstoff, Rutin, Kojisäure, Kaffeesäure, β-Hydroxysäuren, α-Hydroxysäuren, Retinoesäure und ihren Derivaten, Benzol-1,4-di(3-methylen-10-camphersulfonsäure), Pflanzenextrakten, die diese Säuren enthalten, und deren Gemischen ausgewählt ist.

**23.** Zusammensetzung nach einem der Ansprüche 20 bis 22, dadurch gekennzeichnet, daß der Wirkstoff unter Ascorbinsäure, Retinol, Salicylsäure, Milchsäure, Methylmilchsäure, Citronensäure, Mandelsäure, Glucuronsäure, Glykolsäure, Brenztraubensäure, 2-Hydroxybutansäure, 2-Hydroxypentansäure, 2-Hydroxyhexansäure, 2-Hydroxyheptansäure, 2-Hydroxyoctansäure, 2-Hydroxynonansäure, 2-Hydroxydecansäure, 2-Hydroxyundecansäure, 2-Hydroxydodecansäure, 2-Hydroxytetradecansäure, 2-Hydroxyhexadecansäure, 2-Hydroxyoctadecansäure, 2-Hydroxytetracosansäure, 2-Hydroxyeicosansäure, Benzylsäure, Phenylmilchsäure, Gluconsäure, Galacturonsäure, Aleuritsäure, Ribonsäure, Tartronsäure, Weinsäure, Äpfelsäure, Fumarsäure und deren Derivaten und Gemischen ausgewählt ist.

**24.** Zusammensetzung nach einem der Ansprüche 20 bis 23, dadurch gekennzeichnet, daß sie als Wirkstoff Ascorbinsäure und Retinol enthält.

**25.** Zusammensetzung nach einem der Ansprüche 20 bis 24, dadurch gekennzeichnet, daß der Wirkstoff in einer Konzentration im Bereich von 0,01 bis 20 % und vorzugsweise 0,1 bis 10 % des Gesamtgewichts der Zusammensetzung vorliegt.

**26.** Zusammensetzung nach einem der Ansprüche 20 bis 25, dadurch gekennzeichnet, daß sie mindestens einen lipophilen oder hydrophilen Zusatzstoff enthält, der unter den Konservierungsmitteln, Antioxidantien, Maskierungsmitteln, Lösemitteln, Parfums, Füllstoffen, Filtern, Geruchsabsorbern, Färbemitteln, hydrophilen oder lipophilen Wirkstoffen und Lipidvesikeln ausgewählt ist.

**27.** Kosmetische Verwendung der Zusammensetzung nach einem der Ansprüche 20 bis 26 zur Reinigung und/oder zur Behandlung und/oder zum Schutz der Haut und/oder der Schleimhäute und/oder der Keratinfasern.

**28.** Verwendung der Zusammensetzung nach einem der Ansprüche 20 bis 26 zur Herstellung einer dermatologischen Zusammensetzung, die zur Reinigung und/oder zur Behandlung und/oder zum Schutz der Haut und/oder der Schleimhäute und/oder der Keratinfasern bestimmt ist.

**29.** Kosmetisches Verfahren zur Reinigung und/oder zur Behandlung und/oder zum Schutz der Haut und/oder der Keratinfasern, dadurch gekennzeichnet, daß es darin besteht, eine Zusammensetzung nach einem der Ansprüche 20 bis 26 auf die Haut, die Schleimhäute und/oder Keratinfasern aufzutragen.

## Claims

**1.** Water/oil/water triple emulsion comprising by gelled outer aqueous phase and an oily phase constituting, with an inner aqueous, phase, a water/oil primary emulsion, chàracterized in that the oily phase comprises an emulsifier chosen from alkyl dimethicone copolyols and dimethibone copolyols and in that the outer aqueous phase comprises:

1) at least one emulsifying copolymer composed to a major extent of a monoolefinically unsaturated $C_3$-$C_6$ carboxylic acid monomer or of its anhydride and to a minor extent of acrylic acid fatty ester monomer, and
2) at least one crosslinked poly(acrylamidomethylpropanesulphonic acid) comprising, distributed randomly:

a) from 90 to 99.9% by weight of units of following general formula (III):

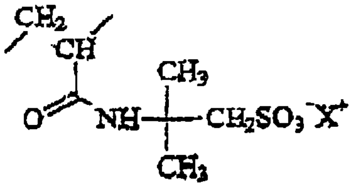 (III)

in which $X^+$ denotes cation or a mixture of cations,
b) from 0.01 to 10% by weight of crosslinking units originating from at least one monomer having at least two olefinic double bonds;

the proportions by weight being defined with respect to the total weight of the polymer.

**2.** Emulsion according to Claim 1, characterized in that the amount of carboxylic acid monomer or of its anhydride in the emulsifying copolymer varies from 80 to 98% by weight and in that the amount of ester monomer varies from 20 to 2% by weight, the percentages by weight being expressed with respect to the total weight of the two monomers.

**3.** Emulsion according to Claim 1 or 2, characterized in that the carboxylic acid monomer is a compound of formula (I):

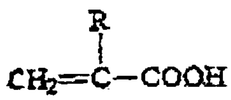 (I)

where R denotes hydrogen, a halogen, a hydroxyl group, a lactone group, a lactam group, a cyanogen group, a monovalent alkyl group, an aryl group, an alkylaryl group, an aralkyl group or a cycloaliphatic group, and in that the ester monomer is a compound of formula (II):

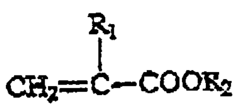 (II)

where $R_1$ is chosen from the group formed by hydrogen, a methyl radical and an ethyl radical and $R_2$ is a $C_8$-$c_{30}$ alkyl group.

**4.** Emulsion according to Claim 3, characterized in that the carboxylic acid monomer is chosen from acrylic acid, methacrylic acid or their mixtures and in that the ester monomer is chosen from monomers of formula (IX) in which $R_1$ is hydrogen or the methyl radical and $R_2$ is a $C_{10}$-$C_{22}$ alkyl group.

**5.** Emulsion according to any one of the preceding claims, characterized in that the emulsifying, copolymer is present in an amount ranging from 0.05 to 3% and preferably from 0.1 to 1% of the total weight of the emulsion.

**6.** Emulsion according to any one of the preceding claims, characterized in that the crosslinked poly(2-acrylamido-2-methylpropanesulphonic acid) comprises from 98 to 99.5% by weight of units of formula (III) and from 0.2 to 2% by weight of crosslinking units.

**7.** Emulsion according to any one of the preceding claims, characterized in that the crosslinked poly(2-acrylamido-2-methylpropanesulphonic acid) is neutralized to at least 90%.

**8.** Emulsion according to any one of the preceding claims, characterized in that, in the formula (III), the $X^+$ cation is $NH_4^+$.

**9.** Emulsion according to any one of the preceding claims, characterized in that the crosslinking monomers correspond to the following general formula (IV) :

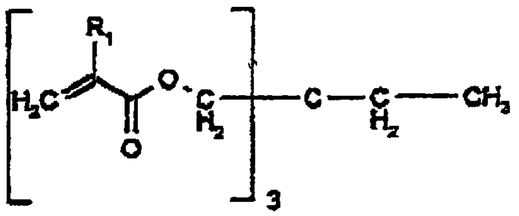

(IV)

in which $R_1$ denotes a hydrogen atom or a $C_1$-$C_4$ alkyl.

**10.** Emulsion according to any one of the preceding claims, characterized in that the poly(2-acrylamido-2-methylpropanesulphonic acid) is crosslinked by trimethylolpropane triacrylate.

**11.** Emulsion according to any one of the preceding claims, characterized in that the crosslinked poly(2-acrylamido-2-methylpropanesulphonic acid) exhibits a viscosity, measured on a Brookfield viscometer, rotor 4, at a rotational speed of 100 revolutions/minute in a 2% solution in water at 25°C, of greater than or equal to 1 Pa.s, preferably ranging from 5 to 40 Pa.s.

**12.** Emulsion according to any one of the preceding claims, characterized in that the crosslinked poly(2-acrylamido-2-methylpropanesulphonic acid) is present in a concentration ranging from 0.01 to 10%, preferably from 0,5 to 2,5%, of the total weight of the composition.

**13.** Emulsion, according to any one of the preceding claims, characterized in that the oily phase comprises at least one silicone oil in an amount ranging from 0.5 to 40% of the total weight of the emulsion.

**14.** Emulsion according to the preceding claim, characterized in that the silicone oil is chosen from the group consisting of volatile silicones, polydimethylsiloxanes, polyphenyltrimethylsiloxanes and fluorinated silicones.

**15.** Emulsion according to any one of the preceding claims, characterized in that the emulsifying agent is present in an amount ranging from 0.01 to 10% and preferably from 0.05 to 5% of the total weight of the emulsion.

**16.** Emulsion according to any one of the preceding claims, characterized in that the oily phase additionally contains one or more other fatty substances chosen from waxes, silicone gums, silicone resins, fluorinated oils, oils of animal origin, oils of plant origin, mineral oils and synthetic oils.

**17.** Emulsion according to any one of the preceding claims, characterized in that the water/oil primary emulsion represents from 10 to 40% and preferably from 20 to 30% of the total weight of the emulsion.

**18.** Emulsion according to any one of the preceding claims, characterized in that the water/oil primary emulsion contains at least one polyol.

**19.** Emulsion according to the preceding claim, characterized in that the polyol or polyols are present in an amount ranging from 0.5 to 50% and preferably from 25 to 45% of the weight of the primary emulsion.

**20.** Topical composition, characterized in that it contains an emulsion according to any one of the preceding claims and at least one active principle.

**21.** Composition according to the preceding claim, characterized in that the active principle is chosen from the group consisting of water-sensitive and/or oxygen-sensitive active principles and acidic active principles.

**22.** Composition according to Claim 20 or 21, characterized in that the active principle is chosen from enzymes, natural extracts, procyanidol oligomers, vitamins, phosphate-comprising and glucosylated derivatives, urea, rutin, kojic acid, caffeic acid, β-hydroxy acids, α-hydroxy acids, retinoic acid and its derivatives, benzene-1,4-di(3-methylidene-10-camphorsulphonic acid), plant extracts comprising such an acid, and their mixtures.

**23.** Composition according to any one of Claims 20 to 22, characterized in that the active principle is chosen from ascorbic acid, retinol, salicylic acid, lactic acid, methyllactic acid, citric acid, mandelic acid, glucuronic acid, glycolic acid, pyruvic acid, 2-hydroxybutanoic acid, 2-hydroxypentanoic acid, 2-hydroxyhexanoic acid, 2-hydroxyheptanoic acid, 2-hydroxyoctanoic acid, 2-hydroxynonanoic acid, 2-hydroxydecanoic acid, 2-hydroxyundecanoic acid, 2-hydroxydodecanoic acid, 2-hydroxytetradecanoic acid, 2-hydroxyhexadecanoic acid, 2-hydroxyoctadecanoic acid, 2-hydroxytetraecosanoic acid, 2-hydroxyeicosanoic acid, benzilic acid, phenyllactic acid, gluconic acid, galacturonic acid, aleuritic acid, ribonic acid, tartronic acid, tartaric acid, malic acid, fumaric acid, their derivatives and their mixtures.

**24.** Composition according to any one of Claims 20 to 23, characterized in that it comprises, as active principles, ascorbic acid and retinol.

**25.** Composition according to any one of Claims 20 to 24, characterized in that the active principle is present in a concentration ranging from 0.01 to 20% and preferably from 0.1 to 10% of the total weight of the composition.

**26.** Composition according to any one of Claims 20 to 25, characterized in that it comprises at least one lipophilic or hydrophilic adjuvant chosen from preservatives, antioxidants, sequestering agents, solvents, fragrances, fillers, screening agents, odour absorbers, colouring materials, hydrophilic or lipophilic active principles and lipid vesicles.

**27.** Cosmetic use of the composition according to any one of Claims 20 to 26 for cleaning and/or treating and/or protecting the skin and/or mucous membranes and/or keratinous fibres.

**28.** Use of the composition according to any one of Claims 20 to 26 for the preparation of a dermatological composition intended for cleaning and/or treating and/or protecting the skin and/or mucous membranes and/or keratinous fibres.

**29.** Cosmetic process for cleaning and/or treating and/or protecting the skin and/or keratinous fibres, characterized in that it consists in applying a composition according to any one of Claims 20 to 26 to the skin, mucous membranes and/or keratinous fibres.